(19) Europäisches Patentamt
European Patent Office
Office européen des brevets

(11) Veröffentlichungsnummer : **0 624 590 A1**

(12) **EUROPÄISCHE PATENTANMELDUNG**

(21) Anmeldenummer : **94810239.7**

(22) Anmeldetag : **28.04.94**

(51) Int. Cl.[5] : **C07D 498/22**, C07D 309/12, A61K 31/55, // (C07D498/22, 311:00, 273:00, 209:00, 209:00, 209:00)

(30) Priorität : **07.05.93 CH 1406/93**
**04.02.94 CH 335/94**

(43) Veröffentlichungstag der Anmeldung :
**17.11.94 Patentblatt 94/46**

(84) Benannte Vertragsstaaten :
**AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Wacker, Oskar, Dr.**
**Löwenbergstrasse 60**
**CH-4059 Basel (CH)**

(54) **N-Acylierte Staurosporinderivate, Verfahren und Zwischenprodukte zu ihrer Herstellung und ihre Verwendung als Arzneimittel.**

(57) Beschrieben sind N-(Tetrahydropyran-4-yloxy-alkanoyl)-staurosporinderivate der Formel I,

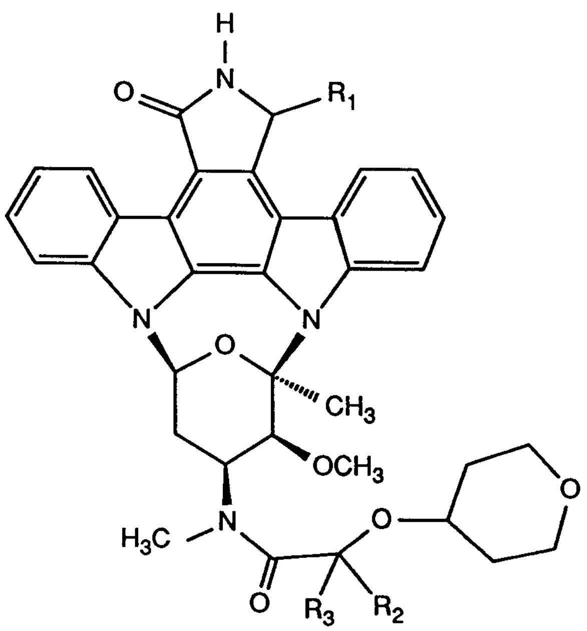

(I)

worin $R_1$ Wasserstoff, Hydroxy, Niederalkoxy oder Oxo, $R_2$ Wasserstoff oder $C_{1-4}$-Alkyl und $R_3$ Wasserstoff oder $C_{1-4}$-Alkyl bedeuten, Verfahren und neue Zwischenprodukte zu ihrer Herstellung und Verfahren zur Herstellung der Zwischenprodukte. Die Verbindungen der Formel I hemmen mit einem hohen Grad an Selektivität das Enzym Proteinkinase C und können insbesondere als tumorhemmende Wirkstoffe verwendet werden.

EP 0 624 590 A1

Die Erfindung betrifft N-acylierte Staurosporinderivate, nämlich N-(Tetrahydropyran-4-yloxy-alkanoyl)-staurosporinderivate, Verfahren und Zwischenprodukte zu ihrer Herstellung, pharmazeutische Präparate, welche diese Verbindungen enthalten, deren Verwendung als Arzneimittel und Verfahren zur Herstellung der Zwischenprodukte.

Staurosporin als Grundstoff der erfindungsgemässen Derivate wurde bereits im Jahre 1977 aus den Kulturen von Streptomyces staurosporeus AWAYA, TAKAHASHI and OMURA, sp. nov. AM 2282, vgl. S. Omura et al., J. Antibiot. 30, 275-281 (1977), isoliert. Bisher war nur die relative, nicht aber die absolute Konfiguration von Staurosporin bekannt. Die absolute Konfiguration wurde erst kürzlich von N. Funato et al., Tetrahedron Letters 35:8, 1251-1254 (1994) publiziert und entspricht dem Spiegelbild der Struktur, die bisher in der Literatur verwendet wurde, um die relative Konfiguration von Staurosporin anzugeben. Dementsprechend wird in der Tetrahedron Letters Publikation wörtlich empfohlen, "that the stereochemical notation for staurosporine which has been in common use hitherto should be revised". Die absolute Konfiguration war zwar bisher nicht bekannt, aber durch die Bezeichnung als "Staurosporinderivat" eindeutig festgelegt. Daher werden, um Verwirrungen beim Vergleich mit den Prioritätsanmeldungen zu vermeiden, in der vorliegenden Anmeldung die ursprünglichen Formeln weiterverwendet.

Staurosporin weist eine starke Hemmwirkung auf Proteinkinase C auf, hemmt aber ebenso stark auch andere Proteinkinasen und hat deshalb nicht die Selektivität, welche für eine therapeutische Anwendung notwendig wäre. Durch übliche Acylreste, wie Benzoyl, substituierte Staurosporinderivate sind zwar selektiver, jedoch sind diese N-acylierten Staurosporinderivate in der Regel relativ schwer löslich und können deshalb nicht leicht zu geeigneten pharmazeutischen Darreichungsformen formuliert werden.

Ziel der vorliegenden Erfindung war die Bereitstellung neuer Staurosporinderivate, die zwar die Hemmaktivität des Staurosporins gegenüber Proteinkinase C (PKC), insbesondere den "konventionellen" Isotypen $\alpha$, $\beta$-1, $\beta$-2 und $\gamma$ von Proteinkinase C, hauptsächlich gegenüber PKC-$\alpha$ und PKC-$\gamma$, behalten, aber gegenüber anderen Proteinkinasen und anderen Isotypen von Proteinkinase C wesentlich weniger wirksam sind. Ausserdem sollten die bereitzustellenden Staurosporinderivate bei oraler Applikation hochwirksam und so gut löslich sein, dass sie ohne grössere Probleme zu geeigneten pharmazeutischen Darreichungsformen formuliert werden können.

Die Erfindung betrifft insbesondere N-(Tetrahydropyran-4-yloxy-alkanoyl)-staurosporinderivate der Formel I,

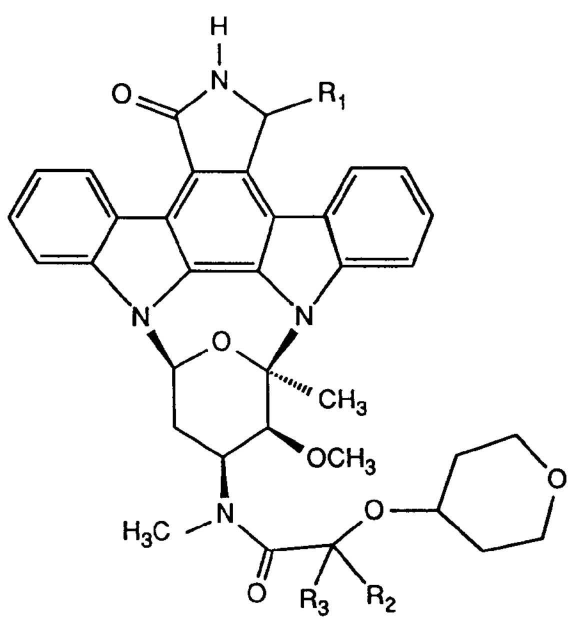

(I)

worin $R_1$ Wasserstoff, Hydroxy, Niederalkoxy oder Oxo, $R_2$ Wasserstoff oder $C_{1-4}$-Alkyl und $R_3$ $C_{1-4}$-Alkyl oder vorzugsweise Wasserstoff bedeuten, Verfahren und Zwischenprodukte zu ihrer Herstellung, pharmazeutische Präparate, welche diese Verbindungen enthalten, deren Verwendung als Arzneimittel und Verfahren zur Herstellung der Zwischenprodukte.

Die aus Formel I ersichtlichen Konfigurationen dienen nur zur Angabe der relativen, nicht aber der absoluten Stereochemie. Wie oben ausgeführt, wird die absolute Stereochemie wahrscheinlich durch die nachste-

hende Formel Ia wiedergegeben.

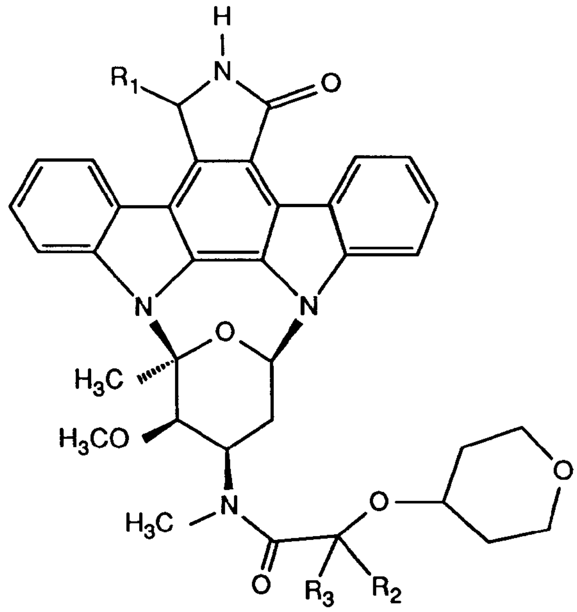

(Ia)

Die Konfiguration am C-$R_2$-Atom ist (D) oder (L), vorzugsweise (D).

Niederalkoxy $R_1$ ist $C_1$-$C_7$-Alkoxy, vorzugsweise $C_{1-4}$-Alkoxy, in erster Linie Methoxy.

$C_{1-4}$-Alkyl $R_2$ oder $R_3$ ist vorzugsweise Methyl.

Die Verbindungen der Formel I besitzen wertvolle pharmakologische Eigenschaften, z.B. hemmen sie mit einem hohen Grad an Selektivität das Enzym Proteinkinase C. Die von Phospholipiden und Calcium abhängige Proteinkinase C kommt innerhalb der Zelle in mehreren Formen vor und beteiligt sich an verschiedenen fundamentalen Vorgängen, wie Signalübertragung, Proliferation und Differenzierung, sowie auch Ausschüttung von Hormonen und Neurotransmittern. Die Aktivierung dieses Enzyms erfolgt entweder durch eine über Rezeptoren vermittelte Hydrolyse von Phospholipiden der Zellmembran oder durch eine direkte Interaktion mit gewissen Tumor-fördernden Wirkstoffen. Die Empfindlichkeit der Zelle gegenüber der rezeptorenvermittelten Signalübertragung kann durch die Abwandlung der Aktivität von Proteinkinase C (als Signalüberträger) wesentlich beeinflusst werden. Verbindungen, die fähig sind, die Aktivität der Proteinkinase C zu beeinflussen, können Anwendung als tumorhemmende, entzündungshemmende, immunomodulierende und antibakterielle Wirkstoffe finden und sogar als Mittel gegen Atherosklerose und Krankheiten des kardiovaskulären Systems und zentralen Nervensystems von Interesse sein.

Zur Bestimmung der Proteinkinase-C-Hemmwirkung verwendet man Proteinkinase C aus Schweinehirn, welche gemäss der von T. Uchida und C.R. Filburn in J. Biol. Chem. 259, 12311-4 (1984) beschriebenen Verfahrensweise gereinigt wird. Die Bestimmung der Proteinkinase-C-Hemmwirkung der Verbindungen der Formel I erfolgt nach der Methodik von D. Fabbro et al., Arch. Biochem. Biophys. 239, 102-111 (1985). In diesem Test hemmen die Verbindungen der Formel I die Proteinkinase C bereits bei einer Konzentration $IC_{50}$ zwischen etwa 0,01 und 0,05 μMol/Liter. Demgegenüber hemmen die Verbindungen der Formel I andere Enzyme, z.B. Proteinkinase A und Protein-Tyrosin-Kinase, erst bei einer weitaus, z.B 100fach höheren Konzentration. Dies zeigt die Selektivität der Verbindungen der Formel I.

Die im obigen Versuch verwendete Proteinkinase C aus Schweinehirn ist ein Gemisch verschiedener Subtypen (Isotypen) von Proteinkinase C. Wenn anstatt Proteinkinase C aus Schweinehirn reine, rekombinante Isotypen im obigen Versuch eingesetzt werden, zeigt sich, dass die Verbindungen der Formel I bevorzugt die konventionellen" Isotypen α, β-1, β-2 und γ hemmen, während die "nicht konventionellen" Isotypen δ, ε und η und die "atypische" Isoform ζ deutlich schwächer bis praktisch gar nicht gehemmt werden.

Rekombinante PKC Isotypen werden folgendermassen kloniert, exprimiert und gereinigt:

Die Herstellung von unterschiedlichen Proteinen mit Hilfe von Baculoviren, deren Klonierung und Isolation aus Sf9 Insektenzellen wird durchgeführt, wie beschrieben von M.D. Summers und G.E. Smith, "A manual method for baculovirus vectors and insect cell culture procedure", Texas Agricul. Exptl. Station Bull. (1987), 1555. Die Konstruktion und die Isolation rekombinanter Viren für die Expression von PKC-α (Rind), PKC-β1 (Mensch), PKC-β2 (Mensch) sowie PKC-γ (Mensch/Rind-Hybrid) in Sf9 Zellen erfolgt wie von Stabel et al. beschrieben [S. Stabel, M. Liyanage und D. Frith, "Expression of protein kinase C isozymes in insect cells and isolation of recombinant proteins", Meth. Neurosc. (1993)]. Die Herstellung der PKC Isotypen in Sf9 Zellen erfolgt wie von Stabel et al. (siehe oben) angegeben, und die Reinigung der Enzyme wird nach der in der Publikation von McGlynn et al. beschriebenen Methode ausgeführt [E. McGlynn, J. Liebetanz, S. Reutener, J. Wood, N.B. Lydon, H. Hofstetter, M. Vanek, T. Meyer und D. Fabbro, "Expression and partial characterization of rat protein kinase C-δ and protein kinase C-ζ in insect cells using recombinant baculovirus", J. Cell. Biochem. 49, 239-250 (1992)]. Für die Generierung rekombinanter PKC-δ (Ratte), PKC-ε (Ratte), PKC-ζ (Ratte) und PKC-η (Maus), deren Expression und Reinigung wird das von Liyanage et. al. ["Protein kinase C group B members PKC-δ, -ε, -ζ and PKC-λ: Comparison of properties of recombinant proteins in vitro and in vivo", Biochem. J. 283, 781-787 (1992)] bzw. McGlynn et. al. (siehe oben) beschriebene Vorgehen befolgt mit dem Zusatz, dass für die Expression von PKC-η der Transfer Vektor pAc360 verwendet wird [V. Luckow und M.D. Summers, "Trends in the development of baculovirus expression", Biotechnology 6, 47-55 (1988)].

Die Messung der Aktivität der nach obiger Methode erhaltenen, rekombinanten PKC Isotypen wird in Abwesenheit von Lipid und Calcium (Co-Faktoren) durchgeführt Hierzu wird Protaminsulfat, welches in Abwesenheit von Co-Faktoren phosphoryliert wird, als Substrat eingesetzt. Die Aktivität der Enzyme reflektiert den Transfer von $^{32}P$ aus γ-[$^{32}P$]-ATP auf Protaminsulfat. Protaminsulfat ist eine Mischung von Polypeptiden, die jeweils vier C-terminale Argininreste enthalten. Die Messung der Phosphat-Inkorporation wird unter folgenden Bedingungen durchgeführt: 100 μl des Reaktionsgemisches enthalten in finalen Konzentrationen 20 mM TRIS-HCl pH 7,4, 10 mM $Mg[NO_3]_2$, 0,5 mg/ml Protaminsulfat, 10 μM ATP (0,1 μCi γ-[$^{32}P$]-ATP; 10 Ci/mol; Amersham, Little Chalfont, United Kingdom), verschiedene Konzentrationen der Hemmsubstanzen und 0,5-2,5 U (Einheiten; eine Einheit ist die Enzymmenge, die in einer Minute pro Milligramm Protein ein Nanomol $^{32}P$ vom obengenannten γ-[$^{32}P$]-ATP auf Histon H1 [Sigma, Typ V-S] überträgt) der Enzyme. Die Reaktion wird durch Zugabe der Enzyme und Transfer auf 32 °C gestartet. Die Reaktionszeit beträgt 20 Minuten. Danach wird die Reaktion gestoppt, indem Aliquots von 50 μl auf P81 Chromatographiepapier (Whatman, Maidstone, United Kingdom) aufgetropft werden. Nach der Entfernung von ungebundenem γ-[$^{32}P$]-ATP und Bruchteil-Nukleotiden durch Waschgänge wie beschrieben von J.J. Witt und R. Roskoski, "Rapid protein kinase assay using phosphocellulose-paper absorption", Anal. Biochem. 66, 253-258 (1975), wird die Substratphosphorylierung durch Szintillationsmessung bestimmt. In diesem Test hemmen die Verbindungen der Formel I die verschiedenen Isotypen der Proteinkinase C (PKC) bereits bei einer Konzentration $IC_{50}$ zwischen etwa 0,001 und 0,1 μMol/Liter im Falle von PKC-α und PKC-γ, zwischen etwa 0,01 und 0,08 μMol/Liter im Falle von PKC-β-1 und PKC-β-2, zwischen etwa 0,03 und 10 μMol/Liter im Falle von PKC-δ, PKC-ε und PKC-η und mehr als 4 μMol/Liter im Fall von PKC-ζ.

Wie bereits aufgrund der obengeschilderten Hemmwirkung auf Proteinkinase C erwartet werden kann, weisen die Verbindungen der Formel I antiproliferative Eigenschaften auf, die sich in folgendem anderen Versuch direkt demonstrieren lassen: Dabei wird die Hemmwirkung der Verbindungen der Formel I auf das Wachstum von menschlichen T24 Blasenkarzinomzellen bestimmt. Diese Zellen werden in "Eagle's minimal essential medium", dem 5 % (V/V) fötales Kälberserum zugesetzt sind, in einem befeuchteten Inkubator bei 37°C und 5 Volumenprozent $CO_2$ in der Luft inkubiert. Die Karzinomzellen (1000-1500) werden in 96-Loch-Mikrotiterplatten eingesät und über Nacht unter den obengenannten Bedingungen inkubiert. Die Testsubstanz wird in seriellen Verdünnungen am Tag 1 hinzugefügt. Die Platten werden unter den obengenannten Bedingungen 5 Tage lang inkubiert. Während dieser Zeitspanne durchlaufen die Kontrollkulturen mindestens 4 Zellteilungen. Nach der Inkubation werden die Zellen mit 3,3%iger (G/V) wässriger Glutaraldehydlösung fixiert, mit Wasser gewaschen und mit 0,05%iger (Gewicht(Volumen) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportional ist, mit einem Photometer (Titertek multiskan) bei 665 nm gemessen. Die $IC_{50}$-Werte werden mit einem Computersystem unter Verwendung der Formel

$$\frac{OD_{665}\,(\text{Test})\ \text{minus}\ OD_{665}\,(\text{Anfang})}{OD_{665}\,(\text{Kontrolle})\ \text{minus}\ OD_{665}\,(\text{Anfang})} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt. Die so ermittelten $IC_{50}$-Werte liegen für die Verbindungen der Formel I zwischen etwa 0,01 und 0,9 μMol/Liter, insbesondere zwischen etwa 0,03 und 0,9 μMol/Liter.

Die Antitumorwirkung der Verbindungen der Formel I lässt sich auch in vivo demonstrieren:

Zur Bestimmung der Antitumorwirkung werden weibliche Balb/c Nacktmäuse mit s.c. transplantierten humanen Blasentumoren T24 verwendet. Am Tag 0 werden den Tieren unter peroraler Forene-Narkose ca. 25 mg eines soliden Tumors unter die Haut auf der linken Flanke geschoben und die kleine Schnittwunde mittels Wundklammer geschlossen. Am Tag 6 nach der Transplantation werden die Mäuse randomisiert in Gruppen

à 6 Tiere verteilt und man beginnt mit der Behandlung. Die Behandlung wird 15 Tage durchgeführt mit einmal täglicher peroraler beziehungsweise intraperitonealer Applikation einer Verbindung der Formel I in Dimethylsulfoxid/Tween 80/Natriumchloridlösung in den verschiedenen Dosen. Zweimal pro Woche werden die Tumoren mit einer Schieblehre ausgemessen und das Tumorvolumen berechnet. In diesem Test bewirkt die perorale oder intraperitoneale Verabreichung von 3 mg/kg täglich einer Verbindung der Formel I eine Verringerung des mittleren Tumorvolumens auf 10-15% des Tumorvolumens bei den unbehandelten Kontrolltieren.

Aufgrund der beschriebenenen Eigenschaften können die Verbindungen der Formel I insbesondere als tumorhemmende Wirkstoffe verwendet werden, z.B zur Therapie von Tumoren der Blase und der Haut. Wenn die Verbindungen der Formel I bei der Krebstherapie in Kombination mit anderen Chemotherapeutika verwendet werden, verhindern sie die Bildung von Resistenz (multidrug resistance) oder heben eine bereits gegenüber den anderen Chemotherapeutika vorhandene Resistenz auf. Ausserdem kommen sie für die oben für Proteinkinase C Modulatoren genannten weiteren Anwendungen in Betracht und können insbesondere zur Behandlung von Krankheiten verwendet werden, die auf eine Hemmung der Proteinkinase C ansprechen.

Die Verbindungen der Formel I hemmen bereits bei einer Konzentration $IC_{50}$ von weniger als 0,08 µMol/Liter, auch bestimmte Tyrosin-Kinasen, wie insbesondere die PDGF-Rezeptor-Kinase.

PDGF (Platelet-derived Growth Factor) ist ein sehr häufig vorkommender Wachstumsfaktor, der eine wichtige Rolle sowohl beim normalen Wachstum als auch bei der pathologischen Zellvermehrung spielt, wie bei der Karzinogenese und bei Erkrankungen der glatten Muskelzellen von Blutgefässen, z.B. bei der Atherosklerose und der Thrombose.

Die Hemmung der Proteinkinase C und der PDGF-Rezeptor-Kinase wirkt in diesem Sinne quasi synergistisch in die gleiche Richtung im Hinblick auf die Regulierung des Zellwachstums.

Die Hemmung der PDGF-stimulierten Rezeptor-Tyrosinkinaseaktivität in vitro wird in PDGF Rezeptor Immunkomplexen von BALB/c 3T3 Zellen gemessen, analog wie beschrieben von E. Andrejauskas-Buchdunger und U. Regenass in Cancer Research 52, 5353-5358 (1992). Die oben näher bezeichneten Verbindungen der Formel I hemmen die PDGF-abhängige zellfreie Rezeptorphosphorylierung bei Konzentrationen von weniger als 0,08 µMol/Liter.

Die Hemmung der PDGF-Rezeptor-Tyrosinkinase in der intakten Zelle wird mittels Western Blot Analyse nachgewiesen, ebenfalls analog wie beschrieben von E. Andrejauskas-Buchdunger und U. Regenass in Cancer Research 52, 5353-5358 (1992). In diesem Test wird die Hemmung der Ligand-stimulierten PDGF-Rezeptor-Autophosphorylierung in BALB/c Mauszellen mit Hilfe von Anti-Phosphotyrosin-Antikörper gemessen. Die Verbindungen der Formel I hemmen die Tyrosinkinase Aktivität des PDGF-Rezeptors bei Konzentrationen von 0,005-0,08 µMol/Liter. Diese Verbindungen hemmen ausserdem in Konzentrationen unterhalb von 1,0 µMol/Liter das Zellwachstum einer PDGF-abhängigen Zelllinie, nämlich der BALB/c 3T3 Mausfibroblasten.

Aufgrund der beschriebenen Eigenschaften können Verbindungen der Formel I nicht nur als tumorhemmende Wirkstoffe, sondern auch als Mittel gegen nicht-maligne proliferative Erkrankungen, wie Atherosklerose, Thrombose, Psoriasis, Sklerodermie und Fibrose verwendet werden. Ausserdem kommen sie für die oben für Proteinkinase C Modulatoren genannten weiteren Anwendungen in Betracht und können insbesondere zur Behandlung von Krankheiten verwendet werden, die auf eine Hemmung der PDGF-Rezeptor-Kinase ansprechen.

Bevorzugt sind Verbindungen der Formel I, worin $R_1$ Wasserstoff oder Oxo, $R_2$ Wasserstoff oder $C_{1-4}$-Alkyl und $R_3$ Wasserstoff bedeuten.

Sehr bevorzugt sind Verbindungen der Formel I, worin $R_1$ Wasserstoff oder Oxo, $R_2$ Wasserstoff oder Methyl und $R_3$ Wasserstoff bedeuten.

Hauptsächlich bevorzugt sind die obengenannten Verbindungen der Formel I, welche die (D)-Konfiguration am C-$R_2$-Atom aufweisen.

Am meisten bevorzugt sind die in den Beispielen beschriebenen Verbindungen der Formel I, hauptsächlich N-[O-(Tetrahydropyran-4-yl)-D-lactoyl]-staurosporin.

Die Verbindungen der Formel I werden nach an sich bekannten Verfahren hergestellt. Das erfindungsgemässe Verfahren ist dadurch gekennzeichnet, dass man ein Amin der Formel II,

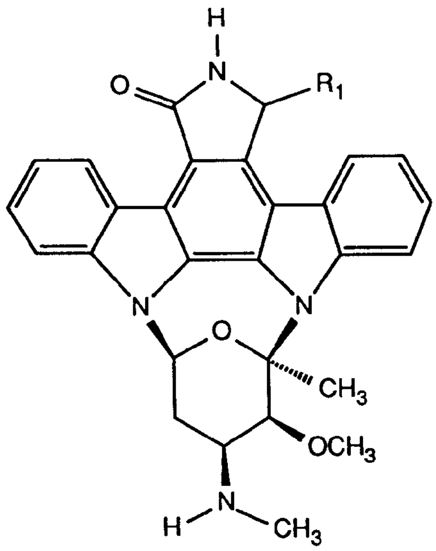

(II)

worin $R_1$ die obengenannte Bedeutung hat mit der Massgabe, dass eine durch $R_1$ repräsentierte Hydroxygruppe erforderlichenfalls durch eine leicht abspaltbare Hydroxyschutzgruppe geschützt ist, mit einer Carbonsäure der Formel III,

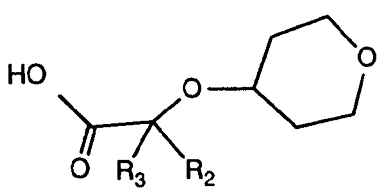

(III)

worin $R_2$ und $R_3$ die obengenannten Bedeutungen haben, oder einem reaktionsfähigen Carbonsäurederivat davon acyliert, und Schutzgruppen, welche im gewünschten Endprodukt der Formel I nicht vorhanden sind, abspaltet und, wenn erwünscht, ein erhaltenes Isomerengemisch auftrennt.

Die Durchführung des obengenannten Verfahrens wird im folgenden näher erläutert:

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitrobenzyloxycarbonyl, oder Diphenylmethoxycarbonyl, oder 2-Halogenniederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa-oder 2-thia-aliphatische oder -cycloaliphatische Kohlenwasserstoffreste, in erster Linie 1-Niederalkoxyniederalkyl oder 1-Niederalkylthioniederalkyl, z.B. Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Die Abspaltung der Schutzgruppen, die nicht Bestandteil des gewünschten Endprodukts der Formel I sind, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion. Durch gegebenenfalls substituiertes 1-Phenylniederalkyl, z.B. Benzyl, geschütztes Hydroxy wird vorzugsweise durch katalytische Hydrierung, z.B. in Gegenwart eine Palladium-auf-Kohle-Katalysators, freigesetzt. Eine durch 2,2-Dichloracetyl

geschützte Hydroxygruppe wird z.B. durch basische Hydrolyse, eine durch tert.- Niederalkyl oder durch einen 2-oxa- oder 2-thia-aliphatischen oder -cycloaliphatischen Kohlenwasserstoffrest veretherte Hydroxygruppe durch Acidolyse, z.B. durch Behandeln mit einer Mineralsäure oder einer starken Carbonsäure, z.B. Trifluoressigsäure, freigesetzt. Mit einem organischen Silylrest, z.B. Trimethylsilyl, verethertes Hydroxy kann auch mit einem Fluoridanionen liefernden Salz der Fluorwasserstoffsäure, z.B. Tetrabutylammoniumfluorid, freigesetzt werden.

Ein reaktionsfähiges Säurederivat einer Verbindung der Formel III ist insbesondere ein reaktionsfähiger (aktivierter) Ester, ein reaktionsfähiges Anhydrid oder ein reaktionsfähiges cyclisches Amid.

Reaktionsfahige (aktivierte) Ester einer Säure der Formel III sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid oder N-Ethyl-N'-(3-dimethylaminopropyl)-carbodiimid-hydrochlond, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronenanziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexylcarbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenylthioester (die man z.B. durch Behandeln der entsprechenden Säure mit gegebenenfalls, z. B. durch Nitro, substituierten Thiophenolen, u.a. mit Hilfe der Anhydrid- oder Carbodiimid-Methode, erhalten kann; Methode der aktivierten Thiolester), Amino- oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxy-phthalimid oder 1-Hydroxy-benzotriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht aber mit Aminogruppen reagieren).

Anhydride einer Säure der Formel III können symmetrische oder vorzugsweise gemischte Anhydride dieser Säure sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Derivate von Säuren der Formel III, die als Acylierungsmittel verwendet werden, können auch in situ gebildet werden. So kann man z.B. N,N'-di-substituierte Amidinoester in situ bilden, indem man das Gemisch des Ausgangsmaterials der Formel V und der als Acylierungsmittel verwendeten Säure in Gegenwart eines geeigneten N,N-disubstituierten Carbodiimids, z.B. N,N'-Dicyclohexylcarbodiimid, zur Reaktion bringt. Ferner kann man Amino- oder Amidoester der als Acylierungsmittel verwendeten Säuren in Gegenwart des zu acylierenden Ausgangsmaterials der Formel V bilden, indem man das Gemisch der entsprechenden Säure- und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Die Reaktion kann in an sich bekannter Weise durchgeführt werden, wobei die Reaktionsbedingungen in erster Linie davon abhängen, ob und wie die Carboxylgruppe des Acylierungsmittels der Formel III aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30°C bis etwa +150°C, insbesondere von etwa 0°C bis +100°C, vorzugsweise von Raumtemperatur (ca. +20°C) bis +70°C, in einem offenen oder geschlossenen Reaktionsgefäss und/oder in der Atmosphäre eines Inertgases, z.B. Stickstoff. Uebliche Kondensationsmittel sind z.B. Carbodiimide, beispielsweise N,N'-Diethyl-, N,N'-Dipropyl- oder N,N'-Dicyclohexylcarbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Von Vorteil sind wasserlösliche Carbodiimide, wie N-Ethyl-N'-(3-dimethylaminopropyl)-carbodiimid. Uebliche säurebindende Kondensationsmittel sind z.B. Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Gemische von Isomeren können in an sich bekannter Weise, z.B. durch fraktionierte Kristallisation, Chromatographie etc. in die einzelnen Isomeren aufgetrennt werden.

Die Ausgangsstoffe der Formel II sind bekannt oder können nach an sich bekannten Verfahren hergestellt werden. Der Ausgangsstoff der Formel II, worin $R_1$ für Wasserstoff steht, d.h. Staurosporin, ist kommerziell erhältlich und kann durch Fermentation mit dem Stamm Streptomyces staurosporeus hergestellt werden. Dieser Stamm wurde unter der Nummer FERM P-3725 beim Fermentation Research Institute, Japan, im Zusammenhang mit der geprüften japanischen Patentveröffentlichung [Examined Patent Publication, Kokoku] Nr. 57-53076 hinterlegt, die am 11. 11. 1982 veröffentlicht wurde, vgl. S. Omura et al., J. Antibiot. 30, 275-281 (1977). Staurosporinderivate der Formel II, worin $R_1$ von Wasserstoff verschieden ist, sind z.B. beschrieben von I. Takahashi et al., J. Pharmacol. Exp. Ther. 255(3) (1990) 1218-1221 sowie in WO-A-8907-105-A (Anmelderin: Kyowa Hakko Kogyo KK, japanische Priorität Nr. 024571 vom 4. 2. 1988). Verbindungen der Formel I, worin $R_1$ Hydroxy oder Oxo bedeutet, werden auch als Nebenprodukt bei der Synthese von Verbindungen der Formel I, worin $R_1$ Wasserstoff bedeutet, erhalten.

Die Ausgangsstoffe der Formel III sind neu. Eine Säure der Formel III erhält man durch Umsetzung von Tetrahydropyran-4-ol mit einer Säure der Formel IV,

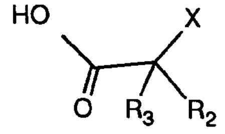

(IV)

worin X für eine nucleophile Abgangsgruppe steht und $R_2$ und $R_3$ die obengenannten Bedeutungen haben. Eine nucleophile Abgangsgruppe X ist insbesondere mit einer geeigneten Mineralsäure, wie einer geeigneten Halogenwasserstoffsäure, oder einer geeigneten Sulfonsäure, wie 4-Toluol-sulfonsäure, verestertes Hydroxy, vorzugsweise Chlor. Dabei wird Tetrahydropyran-4-ol zunächst in einem geeigneten inerten aprotischen Lösungsmittel, wie einem acyclischen oder cyclischen Ether, wie Dioxan, mit einer geeigneten Base, wie Natriumhydrid umgesetzt. Die so erhaltene Suspension wird zu einer Lösung einer Verbindung der Formel IV in einem geeigneten inerten aprotischen Lösungsmittel, wie einem acyclischen oder cyclischen Ether, wie Dioxan, getropft. Die Reaktion wird zwischen 0°C und 150°C, vorzugsweise zwischen 20°C und 100°C, z.B. bei der Rückllusstemperatur des verwendeten Lösungsmittels, durchgeführt.

Die neuen Verbindungen der Formel III und ihre Salze gehören als Zwischenprodukte zur Herstellung der Verbindungen der Formel I ebenfalls zum Gegenstand der Erfindung. Die Verbindungen der Formel III sind überraschend löslich in Wasser und organischen Lösungsmitteln. Die Wasserlöslichkeit bei 22°C beträgt zwischen 100 g und 500 g/Liter. Es ist deshalb denkbar, dass die entsprechenden Acylreste der Verbindungen der Formel m massgeblich für die im Vergleich zu anderen N-Acyl-staurosporinderivaten, wie N-Benzoyl-staurosporin, mehrfach, z.B. mehr als 10fach, erhöhte Löslichkeit der Verbindungen der Formel I in Wasser und anderen Lösungsmitteln verantwortlich sind.

Bevorzugt sind Verbindungen der Formel III, worin $R_2$ Wasserstoff oder Methyl und $R_3$ Wasserstoff oder Methyl bedeuten, in erster Linie die in den Beispielen beschriebenen Verbindungen der Formel III und ihre Salze.

Salze von Verbindungen der Formel III sind in erster Linie Metall- oder Ammoniumsalze, wie Alkalimetall- oder Erdalkalimetallsalze, z.B. Natrium-, Kalium-, Magnesium- oder Calciumsalze oder Ammoniumsalze mit Ammoniak oder geeigeten organischen Aminen, wie tertiären Monoaminen, z.B. Triethylamin oder Tri-(2-hydroxyethyl)-amin, oder heterocyclischen Basen, z.B. N-Ethyl-piperidin oder N,N'-Dimethyl-piperazin.

Die Erfindung betrifft ausserdem das oben beschriebene Verfahren zur Herstellung der neuen Verbindungen der Formel III.

Die oben beschriebenen Verfahren, inklusive die Verfahren zur Abspaltung von Schutzgruppen und die zusätzlichen Verfahrensmassnahmen, werden, wenn nicht anders angegeben, in an sich bekannter Weise, z.B. in An- oder Abwesenheit von vorzugsweise inerten Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa 150°C, insbesondere von etwa 0°C bis etwa +70°C, vorzugsweise von etwa +10°C bis etwa +50°C, hauptsächlich bei Raumtemperatur, in einem geeigneten Gefäss und erforderlichenfalls in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden. Die Erfindung betrifft auch die Verwendung der Verbindungen der Formel I, vorzugsweise in Form pharmazeutischer Zusammensetzungen, zur therapeutischen Behandlung des menschlichen oder tierischen Körpers, insbesondere im Falle der obengenannten Krankheiten. Die Erfindung betrifft auch ein Verfahren zur Hemmung der Proteinkinase C in einem Warmblüter, der einer solchen Behandlung bedarf, wobei man diesem Warmblüter eine effektive Proteinkinase C hemmende Dosis einer Verbindung der Formel I verabreicht. Die Dosierung des Wirkstoffs hängt unter anderem von der Art der Krankheit, der Art und Grösse der zu behandelnden Spezies, der Abwehrlage des Organismus und der Applikationsweise ab. Beispielsweise verabreicht man an einen Warmblüter von etwa 70 kg Körpergewicht eine tägliche Dosis von 1 mg bis 1500 mg, hauptsächlich von 100 mg bis 1000 mg, vorzugsweise von 200 mg bis 800 mg, z.B. 500 mg, einer Verbindung der Formel I. Diese Gesamt-Tagesdosis wird vorzugsweise auf 2-3 tägliche Applikationen verteilt. Dabei liegt die Dosierung bei oraler Applikation etwa zwei- bis dreimal höher als bei parenteraler Applikation, also eher im oberen Bereich der angegebenen Dosierungen.

Die Erfindung betrifft auch pharmazeutische Präparate, die eine wirksame Menge, insbesondere eine zur Prophylaxe oder Therapie einer der obengenannten Krankheiten wirksame Menge, der Aktivsubstanz zusammen mit pharmazeutisch verwendbaren Trägerstoffen enthalten, die sich zur topischen, enteralen, z.B. oralen oder rektalen, oder parenteralen Verabreichung eignen, und anorganisch oder organisch, fest oder flüssig sein können. Zur oralen Verabreichung verwendet man insbesondere Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmacksstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 0,01 % bis 90 %, im Fall von Lyophilisaten bis 100 %, insbesondere von etwa 0,1 % bis etwa 50 %, in erster Linie

zwischen 1 % und 30 %, des bzw. der Aktivstoffe, wobei eine Wirkstoffkonzentration unterhalb von 1% insbesondere für topisch zu applizierende Präparate geeignet ist.

Die nachfolgenden Beispiele illustrieren die Erfindung, ohne sie in irgendeiner Form einzuschränken. Die $R_f$-Werte werden auf Kieselgeldünnschichtplatten (Firma Merck, Darmstadt, Deutschland) ermittelt. Das Verhältnis der Laufmittel in den verwendeten Laufmittelgemischen zueinander ist in Volumenanteilen (V/V), Temperaturen sind in Grad Celsius angegeben. Die Konzentration, c, der Substanz im Lösungsmittel(gemisch) ist im Falle der optischen Drehung in Prozent (Gewicht/Volumen) angegeben.

Beispiel 1:

Zu einer Lösung von 1,13 g (6,5 mMol) O-Tetrahydropyran-4-yl-D-milchsäure in 75 ml absolutem N,N'-Dimethylformamid gibt man bei 0° 1,17 g (7,8 mMol) 1-Hydroxybenzotriazol und 1,61 g (7,8 mMol) N,N'-Dicyclohexylcarbodiimid und rührt die so erhaltene klare, farblose Lösung 3 Stunden bei 0°. Anschliessend werden 2,33 g (5,01 mMol) Staurosporin zugegeben und die so erhaltene farblose Suspension 1 Stunde bei 0° und 20 Stunden bei Raumtemperatur gerührt. Danach gibt man zwecks vollständigem Umsatz des eingesetzten Staurosporins nochmals eine Aktivesterlösung aus 0,38 g (2,18 mMol) 0-Tetrahydropyran-4yl-D-milchsäure, 0,39 g (2,60 mMol) 1-Hydroxybenzotriazol und 0,54 g (2,60 mMol) N,N'-Dicyclohexylcarbodiimid in insgesamt 25 ml absolutem N,N'-Dimethylformamid zu und rührt nach 1 Stunde bei 0° weitere 18 Stunden bei Raumtemperatur. Die so erhaltene gelbliche Suspension giesst man auf 300 ml Wasser, rührt 1 Stunde bei Raumtemperatur, nutscht die ausgefallenen Kristalle ab und wäscht mit Wasser nach. Die Wasserphase wird verworfen. Das Nutschgut wird in 130 ml Methylenchlorid suspendiert und 1,5 Stunden bei Raumtemperatur gerührt. Der ausgefallene N,N'-Dicyclohexylharnstoff wird abgenutscht, mit Methylenchlorid nachgewaschen und das Filtrat im Hochvakuum bei 30° zur Trockne eingedampft. Den Rückstand (gelbe Kristalle) reinigt man durch Säulenchromatographie an 300 g Kieselgel (Typ Si60, Merck 9385, 0,040 - 0,063 mm) in Chloroform (25 ml Fraktionen). Die Fraktionen 220 - 305 werden vereinigt und im Hochvakuum bei 30° zur Trockne eingedampft. Nach zweimaliger Umkristallisation des Rückstands (gelbe Kristalle) aus Essigsäureethylester erhält man N-[0-(Tetrahydropyran-4-yl)-D-lactoyl]-staurosporin als leicht gelbliche Kristalle vom Smp. 222 - 223° (ab 220° Sintern), die noch 0,19 Mol Wasser enthalten; $[\alpha]_D^{20}$= + 166,9 ± 2,0° (c = 0,498; Methanol).

Das Ausgangsmaterial erhält man wie folgt:

Stufe 1.1:

Eine Lösung von 3,06 g (2,85 ml, d = 1,074; 29,96 mMol) Tetrahydro-2H-pyran-4-ol (Fluka, pract.) in 100 ml absolutem 1,4-Dioxan versetzt man bei 65° mit 4,8 g (120 mMol) 60%igem Natriumhydrid in Oel (Fluka, pract.). Die so erhaltene graue Suspension rührt man 2 Stunden unter Rückfluss, lässt wieder auf 65° abkühlen und tropft dann eine Lösung von 3,25 g (2,59 ml, d = 1,25; 29,95 mMol) S(-)-2-Chlor-propionsäure (Fluka, puriss.) im 60 ml absolutem 1,4-Dioxan innerhalb von 8 Minuten zu. Die so erhaltene braune Suspension verdünnt man mit 100 ml absolutem 1,4-Dioxan und erhitzt 3 Stunden unter Rückfluss und Rühren. Anschliessend wird noch 14 Stunden bei Raumtemperatur nachgerührt. Die so erhaltene braune Suspension wird nun innerhalb von 2 Minuten tropfenweise mit 40 ml Wasser versetzt und die so erhaltene gelbe Lösung im Hochvakuum zur Trockne eingedampft. Den Rückstand nimmt man in 200 ml Wasser auf und extrahiert die wässrige Lösung je einmal mit 250 und 150 ml Essigsäureethylester. Die Essigesterextrakte werden einmal mit 100 ml Wasser nachgewaschen. Alle Wasserphasen werden vereinigt und dann mit 4normaler Salzsäure angesäuert (pH 1). Diese so erhaltene Lösung wird mit Kochsalz gesättigt und zweimal mit je 300 ml Essigsäureethylester extrahiert. Die organischen Phasen werden dreimal mit je 150 ml gesättigter Kochsalzlösung nachgewaschen. Anschliessend werden alle Essigesterextrakte vereinigt, über Magnesiumsulfat getrocknet, filtriert und im Hochvakuum bei 30° zur Trockne eingedampft. Der Rückstand (gelbes Oel) wird durch Kugelrohrdestillation (Sdp. ca. 160° bei 0,6 mm Hg) gereinigt. Man erhält O-(Tetrahydropyran-4-yl)-D-milchsäure als leicht gelbliches Oel; $[\alpha]_D^{20}$= 46,7 ± 0,9° (c = 1,058; Chloroform). Das Oel kristallisiert aus Essigsäureethyl ester-Hexan 1:1 als farblose Kristalle vom Schmelzpunkt 68,7-69,5°; $[\alpha]_D^{20}$ = + 48,8 ± 0,8° (c = 1; Chloroform).

Beispiel 2:

Aus den Mutterlaugen der Umkristallisationen des Endprodukts von Beispiel 1 isoliert man durch Flashchromatographie bei 0,4 bar an 90 g Kieselgel (Typ Si60, Merck 9385, 0,040 - 0,063 mm) in Methylenchlorid-Methanol (98 : 2; 10 ml Fraktionen) nach Eindampfen zur Trockne im Hochvakuum der Fraktionen 23 - 27 N-[0-(Tetrahydropyran-4-yl)-D-lactoyl]-7-oxo-staurosporin als gelbe Kristalle vom Smp. 206-208° (ohne Umkristallisation), (+)FAB, MS : $(M+H)^+$ = 637, $[\alpha]_D^{20}$= + 138,7 ± 10,8° (c = 0,185; Methanol:Chloroform = 1 : 1).

Beispiel 3:

Zu einer Lösung von 8,16 g (46,9 mMol) O-Tetrahydropyran-4-yl-D-milchsäure in 400 ml absolutem N,N'-Dimethylformamid gibt man bei 0° unter Argon 9,16 g (61,0 mMol) 1-Hydroxybenzotriazol und 11,7 g (61,0 mMol) N-Ethyl-N'-(3-dimethylaminopropyl)-carbodiimid-hydrochlorid (EDC) und rührt die so erhaltene klare, farblose Lösung 3 Stunden bei 0°. Anschliessend werden 17,50 g (37,5 mMol) Staurosporin zugegeben, und die so erhaltene gelbliche Lösung wird 2 Stunden bei 0° und 19 Stunden bei Raumtemperatur gerührt. Danach wird die gelbliche Lösung im Hochvakuum zur Trockne eingedampft. Den Rückstand rührt man 15 Minuten mit 250 ml Wasser, nutscht ab und wäscht die so erhaltenen beigen Kristalle mit Wasser nach. Die Kristalle reinigt man durch Flashchromatographie bei 0,4 bar an 500 g Kieselgel (Typ Si60, Merck 9385, 0,040 - 0,063 mm) in Methylenchlorid - Methanol (98:2; 25 ml Fraktionen). Die Fraktionen 70 - 140 werden vereinigt und im Hochvakuum bei 30° eingedampft. Den Rückstand kristallisiert man aus 400 ml Essigsäureethylester und erhält fast DC-reines N-[O-(Tetrahydropyran-4-yl)-D-lactoyl]-staurosporin als beige Kristalle. Die Fraktionen 45 - 69 und 141 - 170 der oben genannten Flashchromatographie werden ebenfalls vereinigt und im Hochvakuum eingedampft. Die auf diese Weise erhaltenen gelben Kristalle und die gelben Kristalle aus der Mutterlauge der 1. Umkristallisation werden nochmals einer Flashchromatographie an 500 g Kieselgel Si60 unter denselben Bedingungen wie bereits beschrieben unterzogen. Nach Eindampfen der DC-reinen Fraktionen der 2. Flashchromatographie werden diese mit den zuerst gewonnenen beigen Kristallen vereinigt und nochmals aus 800 ml Essigsäureethylester umkristallisiert. Dadurch erhält man N-[O-(Tetrahydropyran-4-yl)-D-lactoyl]-staurosporin als beige Kristalle vom Smp. 220 - 222° (ab 214° Sintern), die noch 0,42 Mol Wasser enthalten; $[\alpha]_D^{20}$ = + 166,6 ± 2,5° (c = 0,404; Methanol).

Beispiel 4:

Analog Beispiel 3 erhält man aus 310 mg (1,78 mMol) O-Tetrahydropyran-4-yl-L-milchsäure, 347 mg (2,31 mMol) 1-Hydroxybenzotriazol, 443 mg (2,31 mMol) N-Ethyl-N'-3(3-dimethylaminopropyl)-carbodiimid-hydrochlorid (EDC) und 664 mg (1,42 mMol) Staurosporin in 15 ml absolutem N,N'-Dimethylformamid nach einer Reaktionszeit von 5 Stunden bei 0° und 16 Stunden bei Raumtemperatur unter Argon und nachfolgender analoger Flashchromatographie N-[O-(Tetrahydropyran4-yl)-L-lactoyl]-staurosporin als beige Kristalle vom Smp. 302 - 304° (ab 280° Sintern; aus Essigsäureethylester); $[\alpha]_D^{20}$ = + 145,6 ± 1,8° (c = 0,544; Chloroform).

Das Ausgangsmaterial erhält man folgendermassen:

Stufe 4.1:

Analog Stufe 1.1 erhält man aus 1,021 g (0,951 ml, d = 1,074; 10 mMol) Tetrahydro-2H-pyran-4-ol (Fluka, pract.), 1,60 g Natriumhydrid (60%ig in Oel, Fluka pract.) und 1,08 g (0,863 ml, d = 1,258; 10 mMol) R(+)-2-Chlorpropionsäure (Fluka, puriss.) in insgesamt 55 ml 1,4-Dioxan nach Eindampfen der Essigesterextrakte und Kugelrohrdestillation des so erhaltenen Rückstands (Sdp. ca. 160°, bei 0,8 mm Hg) O-(Tetrahydropyran-4-yl)-L-milchsäure als farbloses Oel, das beim Stehen zu farblosen Kristallen erstarrt, die zwischen 33,7 und 67,6° schmelzen und noch 0,13 Mol (1,30 %) Wasser enthalten; $[\alpha]_D^{20}$ = -46,7 ± 1,0° (c = 1,035; Chloroform).

Beispiel 5:

Analog Beispiel 3 enthält man aus 200 mg (1,25 mMol) O-Tetrahydropyran-4-yl-glykolsäure, 244 mg (1,62 mMol) 1-Hydroxybenzotriazol, 311 mg (1,62 mMol) N-Ethyl-N'-(3-dimethylaminopropyl)-carbodiimid-hydrochlorid (EDC) und 466 mg (1,0 mMol) Staurosporin in 10 ml absolutem N,N'-Dimethylformamid nach einer Reaktionszeit von 2 Stunden bei 0° und 18 Stdn. bei Raumtemperatur, Flashchromatographie (analog Beispiel 3, jedoch 20 ml Fraktionen, Produkt in den Fraktionen 15-20) und Umkristallisation des so gereinigten Produkts aus Essigsäureethylester-Hexan (1:1) N-[2-(Tetrahydro-pyran-4-yloxy)-acetyl]-staurosporin als beige Kristalle vom Smp. 222 - 224° (ab 215° Sintern), die noch 0,29 Mol Wasser enthalten; $[\alpha]_D^{20}$ = + 180,0 ± 2,0° (c = 0,510; Chloroform), $R_f$ = 0,26 (Methylenchlorid : Ethanol = 95:5); $R_f$ = 0,48 (Aceton); $R_f$ = 0,64 (Methylenchlorid: Methanol = 9:1).

Das Ausgangsmaterial erhält man wie folgt:

Stufe 5.1:

Eine Lösung von 2,042 g (1,902 ml, d = 1,074; 20,0 mMol) Tetrahydro-2H-pyran-4-ol (Fluka, pract.) in 70 ml absolutem 1,4-Dioxan versetzt man bei 65° mit 3,20 g (80 mMol) 60%igem Natriumhydrid in Oel (Fluka, pract.). Die so erhaltene graue Suspension rührt man 3 Stunden unter Rückfluss, lässt wieder auf 65° abkühlen und tropft dann eine Lösung von 1,89 g (20,0 mMol) Chloressigsäure (Fluka, puriss.) in 40 ml absolutem 1,4-Dioxan innerhalb von 20 Minuten zu. Die so erhaltene graubraune Suspension wird anschliessend erneut auf Rückfluss erhitzt und 3 Stunden bei dieser Temperatur nachgerührt. Nach weiteren 16 Stunden Rühren bei Raumtemperatur werden 10 ml Wasser innerhalb von 5 Minuten zugetropft und die so erhaltene gelbliche Suspension im Hochvakuum zur Trockne eingedampft. Den Rückstand nimmt man in 20 ml Wasser auf und extrahiert die wässrige Lösung zweimal mit je 20 ml Essigsäureethylester. Die Essigesterextrakte werden einmal mit 10 ml Wasser nachgewaschen. Die Wasserphasen werden vereinigt und dann mit 4N Salzsäure angesäuert (pH1). Die so erhaltene Lösung wird mit Kochsalz gesättigt und zweimal mit je 50 ml Essigsäureethylester extrahiert. Die Essigesterphasen werden zweimal mit je 20 ml gesättigter Kochsalzlösung nachgewaschen. Anschliessend werden alle Essigesterextrakte vereinigt, über Magnesiumsulfat getrocknet, filtriert und im Hochvakuum bei 30° zur Trockne eingedampft. Der Rückstand (gelbes Oel) wird durch Kugelrohrdestillation (Sdp. ca. 130° bei 0,4 mm Hg) gereinigt. Man erhält O-(Tetrahydropyran-4-yl)-glykolsäure als farbloses Oel, das beim Stehen zu farblosen Kristallen erstarrt, die zwischen 63,9 und 70,6° schmelzen (ab 60,2° Sintern) und 0,08 Mol (0,91 %) Wasser enthalten.

Beispiel 6:

Aus den Fraktionen 9-11 der Flashchromatographie von Beispiel 5 erhält man nach Umkristallisation aus Essigsäureethylester-Hexan 1:1 als Nebenprodukt N-[2-(Tetrahydro-pyran-4-yloxy)-acetyl]-7-oxo-staurosporin als gelbe Kristalle vom Schmelzpunkt 190,7-192,4° (Sintern ab 187°), die noch 0,35 Mol Wasser enthalten; $[\alpha]_D^{20}$ = + 157,4 ± 2,0° (c = 0,491; Chloroform), $R_f$ = 0,35 (Methylenchlorid: Ethanol = 95:5), $R_f$ = 0,63 (Aceton), $R_f$ = 0,73 (Methylenchlorid: Methanol = 9:1).

Beispiel 7:

Aus den Fraktionen 26-36 der Flashchromatographie von Beispiel 5 erhält man als Nebenprodukt in Lösung instabiles N-[2-(Tetrahydropyran-4-yloxy)-acetyl]-7-hydroxy-staurosporin (Diastereomerengemisch) als beige Kristalle vom Smp. 235 - 237 (ab 227° Sintern; aus Essigsäureethylester-Hexan 1:1), die noch 0,69 Mol Wasser enthalten; $[\alpha]_D^{20}$ = + 209,6 ± 2,0° (c = 0,125; Chloroform), $R_f$ = 0,24 (Methylenchlorid : Ethanol = 95:5), $R_f$ = 0,55 (Aceton), $R_f$ = 0,55 (Methylenchlorid : Methanol = 9:1).

Beispiel 8:

Analog Beispiel 3 erhält man aus 1,327 g (7,05 mMol) 2-Methyl-2-(tetrahydropyran-4-yloxy)-propionsäure, 1,376 g (9,16 mMol) 1-Hydroxybenzotriazol, 1,757 g (9,16 mMol) N-Ethyl-N'-(3-dimethylaminopropyl)-carbodiimid-hydrochlorid (EDC) und 2,63 g (5,64 mMol) Staurosporin in 50 ml absolutem N,N'-Dimethylformamid nach einer Reaktionszeit von 24 Stunden bei Raumtemperatur unter Argon, Flashchromatographie bei 0,4 bar (20 ml-Fraktionen) an 500 g Kieselgel (Typ Si60, Merck 9385, 0,040 - 0,063 mm) in Methylenchlorid-Aceton (9:1; Fraktionen 1 - 100) und Methylenchlorid-Aceton (1:1; Fraktionen 100 - 200) N-[2-Methyl-2-(tetrahydropyran-4-yloxy)-propionyl]-staurosporin. Zur weiteren Reinigung werden die Fraktionen 134 - 170 vereinigt und im Hochvakuum bei 30° zur Trockne eingedampft. Der so erhaltene Rückstand wird nochmals durch Flashchromatographie bei 0,4 bar an 100 g Kieselgel (Typ Si60, Merck 9385, 0,040 - 0,063 mm) in Methylenchlorid-Methanol (98:2; 25 ml Fraktionen) gereinigt. Die Fraktionen 21 - 28 werden vereinigt und im Hochvakuum bei 30° eingedampft. Nach Umkristallisation des Rückstands aus 13 ml Essigsäureethylester-Cyclohexan (1:4) erhält man N-[2-Methyl-2-(tetrahydropyran-4-yloxy)-propionyl]-staurosporin als schwach beige Kristalle vom Smp. 209 - 211° (ab 204° Sintern), die noch 0,38 Mol (1,07 %) Wasser enthalten; $[\alpha]_D^{20}$ = + 154,7 ± 2,0° (c = 0,497; Chloroform).

Das Ausgangsmaterial erhält man wie folgt:

Stufe 8.1a:

Zu einer Lösung von 20 ml (40 mMol) Lithiumdiisopropylamid (2molare Lösung in Tetrahydrofuran-Cyclohexan) in 20 ml absolutem Tetrahydrofuran tropft man unter Argon bei 0-9° innerhalb von 15 Minuten eine Lösung von 3,48 g (20 mMol) O-(Tetrahydropyran-4-yl)-D-milchsäure in 20 ml absolutem Tetrahydrofuran. Die so erhaltene rote Lösung wird eine Stunde bei 0° nachgerührt und dann auf -75° abgekühlt. Danach wird innerhalb von 2 Minuten eine Lösung von 2,84 g (1,25 ml, d = 2,280; 20 mMol) Methyliodid in 10 ml absolutem Tetrahydrofuran zugetropft, wobei die Temperatur auf -61° steigt und die Farbe der Lösung nach gelb umschlägt. Diese gelbe Lösung rührt man noch 14 Stunden unter allmählicher Erwärmung auf Raumtemperatur, giesst dann die so erhaltene gelbe Suspension auf 100 ml Eiswasser und extrahiert zweimal mit je 100 ml Essigsäureethylester. Die Essigesterphasen werden einmal mit 50 ml Wasser nachgewaschen. Die Wasserphasen werden vereinigt, mit 4 N Salzsäure angesäuert und zweimal mit je 100 ml Essigsäureethylester extrahiert. Die Essigesterphasen werden zweimal mit je 50 ml Wasser nachgewaschen, vereinigt, über Natriumsulfat getrocknet und im Hochvakuum bei 30° zur Trockne eingedampft. Das Rohprodukt wird in 30 ml Essigsäureethylester gelöst und die so erhaltene Lösung bei Raumtemperatur mit 2,73 ml Dicyclohexylamin (Fluka, puriss.) versetzt. Die langsam ausgefallenen Kristalle werden abgenutscht, mit wenig Essigsäureethylester nachgewaschen und noch zweimal aus je 30 ml Essigester umkristallisiert. Man erhält das Dicyclohexylammoniumsalz der 2-Methyl-2-(tetrahydropyran-4-yloxy)-propionsäure als farblose Kristalle, die zwischen 131,7 und 137,8° schmelzen (ab 128° Sintern).

Das Salz kann direkt zur weiteren Synthese verwendet oder aber folgendermassen zur freien 2-Methyl-2-(tetrahydropyran-4-yloxy)-propionsäure umgesetzt werden. 3,8 g (0,01 Mol) Dicyclohexylammoniumsalz werden in 50 ml Wasser gelöst. Diese Lösung wird mit 4 N Salzsäure auf pH 1 gebracht. Das ausgefallene Dicyclohexylammoniumchlorid wird abgenutscht und mit wenig Wasser nachgewaschen. Die Wasserphase wird dann zweimal mit je 100 ml Essigsäureethylester extrahiert. Die Extrakte werden zweimal mit je 50 ml Wasser gewaschen, vereinigt, über Natriumsulfat getrocknet und im Hochvakuum bei 30° zur Trockne eingedampft. Nach Umkristallisation des Rückstandes aus Cyclohexan erhält man 2-Methyl-2-(tetrahydropyran-4-yloxy)-propionsäure als farblose Kristalle, die zwischen 90,2 und 93,8° (ab 79,5° Sintern) schmelzen.

Alternativ erhält man 2-Methyl-2-(tetrahydropyran4-yloxy)-propionsäure nach einem von H. Gilman und G.R. Wilder in J. Am. Chem Soc. <u>77</u>, 6644 (1955) beschriebenen Verfahren folgendermassen:

<u>Stufe 8.1b:</u>

5,10 g (50 mMol) Tetrahydro-2H-pyran-4-ol (Fluka, pract.) werden in 48,0 g (60 ml, d = 0,79; 826 mMol) absolutem Aceton (Fluka, puriss.) gelöst. Zu dieser Lösung werden bei Raumtemperatur unter Rühren nach und nach 8,11 g (5,45 ml, d = 1,49; 68 mMol) Chloroform (Fluka, puriss.) und 9,60 g (240 mMol) Natriumhydroxid (Merck, p.a.) gegeben, wobei sich das Reaktionsgemisch innerhalb von 10 Minuten von 23° auf 58° (Rücklluss) erwärmt. Die so erhaltene farblose Suspension kühlt nach 30 Minuten bei 58° langsam von selbst wieder ab. Sie wird erneut zum Rückfluss erhitzt und noch 5 Stunden bei dieser Temperatur gerührt. Nach erneutem Abkühlen dampft man im Hochvakuum bei 30° zur Trockne ein. Der Rückstand wird in 50 ml Wasser aufgenommen, mit 4normaler Salzsäure angesäuert (pH 1) und zweimal mit je 100 ml Essigsäureethylester extrahiert. Diese Extrakte wäscht man zweimal mit je 50 ml gesättigter Kochsalzlösung. Die Essigesterphasen werden vereinigt, über Natriumsulfat getrocknet, filtriert und erneut eingedampft. Den Rückstand reinigt man durch Säulenchromatographie an 500 g Kieselgel (Typ Si60, Merck 9385, 0,040-0,063 mm) in Methylenchlorid-Methanol-Wasser 70:30:5 (20 ml Fraktionen). Die Fraktionen 47-80 werden vereinigt und im Hochvakuum bei 30° eingedampft. Der Rückstand (3,10 g; schmierige Kristalle) wird in 30 ml Diethylether suspendiert. Diese Suspension wird 1/2 Stunde bei Raumtemperatur gerührt. Danach werden die so erhaltenen Kristalle abgenutscht und mit Ether nachgewaschen. Das Nutschgut wird in einem Gemisch von 15 ml Wasser und 20 ml Essigsäureethylester aufgenommen, mit 4normaler Salzsäure auf pH 1 gebracht und die Essigesterphase abgetrennt. Nach Waschen der Essigesterphase mit insgesamt 20 ml Wasser werden alle Essigesterphasen vereinigt, über Natriumsulfat getrocknet, filtriert und im Hochvakuum bei 30° eingedampft. Der Rückstand (0,41 g) wird in 10 ml Essigsäureethylester gelöst und diese Lösung mit 0,437 ml Dicyclohexylamin (Fluka, puriss.) versetzt. Man erhält 2-Methyl-2-(tetrahydropyran-4-yloxy)-propionsäure-dicyclohexylammoniumsalz als farblose Kristalle, die zwischen 136,6 und 138,8° schmelzen (ab 130° Sintern) und ebenso wie oben beschrieben zur freien 2-Methyl-2-(tetrahydropyran-4-yloxy)-propionsäure umgesetzt werden können.

Alternativ erhält man 2-Methyl-2-(tetrahydropyran-4-yloxy)-propionsäure-dicyclohexylammoniumsalz auch nach folgendem Verfahren:

<u>Stufe 8.1c:</u>

10,21 g (100 mMol) Tetrahydro-2H-pyran-4-ol (Fluka, pract.) werden in 350 ml absolutem 1,4-Dioxan (Fluka, puriss.) gelöst. Die so erhaltene Lösung wird auf 65° erwärmt. Dann werden bei 65° 12,0 g (300 mMol) Natriumhydrid (60%ig, in Oel; Fluka, pract.) zugegeben. Die so erhaltene graue Suspension wird 3 Stunden

unter Rückfluss gerührt. Danach wird wieder auf 65° abgekühlt und anschliessend innerhalb von 25 Minuten eine Lösung von 16,70 g (100 mMol) α-Bromisobuttersäure (Fluka, pract.) in 150 ml absolutem Dioxan zugetropft. Die so erhaltene Suspension wird noch 3 Stunden unter Rückfluss und danach 17 Stunden bei Raumtemperatur gerührt. Anschliessend werden 25 ml Wasser vorsichtig zugetropft und die gelbe Suspension im Hochvakuum zur Trockne eingedampft. Der Rückstand wird in 50 ml Wasser aufgenommen und zweimal mit je 100 ml Essigsäureethylester extrahiert. Die Extrakte werden einmal mit 50 ml Wasser nachgewaschen. Die Wasserphasen werden vereinigt, mit 4normaler Salzsäure auf pH 1 gebracht, mit Kochsalz gesättigt und zweimal mit je 50 ml Essigester extrahiert. Diese Extrakte werden zweimal mit je 50 ml gesättigter Kochsalzlösung nachgewaschen. Alle Essigesterphasen werden vereinigt, über Natriumsulfat getrocknet, filtriert und im Hochvakuum eingedampft. Den Rückstand (5,22 g, gelbes Oel) reinigt man durch Flashchromatographie (alles 25 ml Fraktionen) bei 0,4 bar an 500 g Kieselgel (Typ Si60, Merck 9385; 0,040-0,063 mm) in Methylenchlorid-Methanol (9:1; Fraktionen 1-50), Methylenchlorid-Methanol (4:1; Fraktionen 51-150) und Methylenchlorid-Methanol (7:3; (Fraktionen 151-225). Die Fraktionen 16-200 werden vereinigt und im Hockvakuum eingedampft. Der Rückstand wird mit 25 ml Diethylether 1/4 Stunde bei Raumtemperatur verrührt. Die so erhaltenen Kristalle werden abgenutscht und mit Diethylether nachgewaschen. Anschliessend werden die farblosen Kristalle in 10 ml Wasser aufgenommen, mit 4normaler Salzsäure auf pH 1 gestellt und zweimal mit je 20 ml Essigsäureethylester extrahiert. Die Essigesterphasen werden nach zweimaligem Waschen mit je 10 ml Wasser vereinigt, über Natriumsulfat getrocknet, filtriert und im Hochvakuum eingedampft. Der Rückstand wird in 10 ml Essigester gelöst und mit 0,317 ml Dicyclohexylamin versetzt. Man erhält 2-Methyl-2-(tetrahydropyran-4-yloxy)-propionsäure-dicyclohexylammoniumsalz als farblose Kristalle, die zwischen 136,5 und 138,8° schmelzen (ab 130° Sintern).

Beispiel 9:

Tabletten enthaltend 20 mg an Wirkstoff, z.B. eine der in den vorangehenden Beispielen beschriebenen Verbindungen der Formel I, werden in folgender Zusammensetzung in üblicher Weise hergestellt:

| Zusammensetzung | |
|---|---|
| Wirkstoff | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der Sfachen Menge Wasser auf dem Wasserbad verkleistert und die Pulvermischung mit diesem Kleister angeknetet, bis eine schwach plastische Masse entstanden ist.

Die plastische Masse wird ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 10: Antitumorwirkung von N-[0-(Tetrahydropyran-4-yl)-D-lactoyl]-staurosporin in vivo:

Die Substanz wird folgendermassen formuliert:
12,5 mg Aktivsubstanz werden in 0,25 ml Dimethylsulfoxid gelöst und mit 50 μl Tween 80 vermischt. Anschliessend werden 4,7 ml einer 0,9 %igen Natriumchloridlösung zugegeben und das Ganze gut gemischt.

Zur Bestimmung der Antitumorwirkung werden weibliche Balb/c Nacktmäuse mit s.c. transplantierten humanen Blasentumoren T24 verwendet. Am Tag 0 werden den Tieren unter peroraler Forene-Narkose ca. 25 mg eines soliden Tumors unter die Haut auf der linken Flanke geschoben und die kleine Schnuttwunde mittel Wundklammer geschlossen. Am Tag 6 nach der Transplantation werden die Mäuse randomisiert in Gruppen à 6 Tiere verteilt und mit der Behandlung begonnen. Die Behandlung wird 15 Tage durchgeführt mit einmal täglicher Applikation der verschiedenen Dosen. Zweimal pro Woche werden die Tumoren mit einer Schieblehre ausgemessen und das Tumorvolumen berechnet. Die Ergebnisse sind in der nachfolgenden Tabelle zusammengestellt. Darin bedeutet "Dosis" die tägliche Dosis, "Appl." die Applikationsart, "Exper." Experiment und "T/C %" den Quotienten in Prozent aus den Werten bei den behandelten (treated) Mäusen und den unbehandelten Kontrollmäusen (Control). Je kleiner dieser Quotient, desto wirksamer ist die verabreichte Dosis.

| Dosis [mg/kg] | Appl. | Mittleres Tumorvolumen [T/C %] | |
|---|---|---|---|
| | | Exper. 1 | Exper. 2 |
| 6,25 | p.o. | 15 | 12 |
| 3,13 | p.o. | 17 | 14 |
| 1,56 | p.o. | | 31 |
| 0,78 | p.o. | | 58 |
| 3,00 | i.p. | 9 | 11 |
| 1,50 | i.p. | 14 | 19 |
| 0,75 | i.p. | | 32 |
| 0,38 | i.p. | | 62 |
| 0,19 | i.p. | | 74 |

Beispiel 11: Ermittlung der maximal tolerierten Dosis (MTD) von N-[0-(Tetrahydropyran-4-yl)-D-lactoyl]-staurosporin

3 weibliche Balb/c Mäuse per Dosis werden i.p. oder p.o. mit N-[0-(Tetrahydropyran-4-yl)-D-lactoyl]-staurosporin in Dimethylsulfoxid/Tween 80/Natriumchloridlösung (Formulierung siehe Beispiel 10) behandelt. Die Dosis wird erhöht bis Tiere innerhalb von 7 Tagen sterben.
MTD (p.o.): 62,5 mg/kg
MTD (i.p.): 31,25 mg/kg

**Patentansprüche**

**1.** N-(Tetrahydropyran-4-yloxy-alkanoyl)-staurosporinderivate der Formel I,

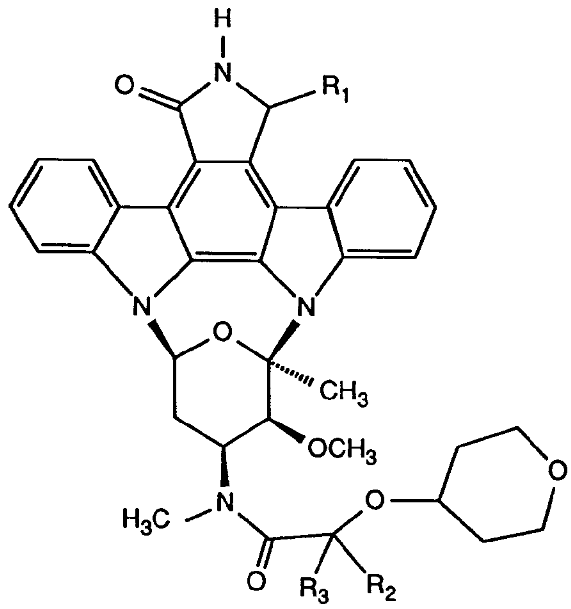

(I)

worin die Konfigurationsangaben nur der Beschreibung der relativen Stereochemie dienen, während die absolute Stereochemie derjenigen von Staurosporin entspricht, und worin $R_1$ Wasserstoff, Hydroxy, Niederalkoxy oder Oxo, $R_2$ Wasserstoff oder $C_{1-4}$-Alkyl und $R_3$ Wasserstoff oder $C_{1-4}$-Alkyl bedeuten.

**2.** Verbindungen nach Anspruch 1 der Formel I, worin $R_3$ Wasserstoff bedeutet.

**3.** Verbindungen nach Anspruch 1 der Formel I, worin $R_1$ Wasserstoff, Hydroxy oder Oxo, $R_2$ Wasserstoff oder $C_{1-4}$-Alkyl und $R_3$ Wasserstoff oder $C_{1-4}$-Alkyl bedeuten.

**4.** Verbindungen nach Anspruch 1 der Formel I, worin $R_1$ Wasserstoff oder Oxo, $R_2$ Wasserstoff oder $C_{1-4}$-Alkyl und $R_3$ Wasserstoff bedeuten.

**5.** Verbindungen nach Anspruch 1 der Formel I, worin $R_1$ Wasserstoff oder Oxo, $R_2$ Wasserstoff oder Methyl und $R_3$ Wasserstoff bedeuten.

**6.** Verbindungen nach einem der Ansprüche 1-5 der Formel I, welche die (D)-Konfiguration am $\underline{C}$-$R_2$-Atom aufweisen, wenn R2 von R3 verschieden ist.

**7.** N-[O-(Tetrahydropyran-4-yl)-D-lactoyl]-staurosporin nach Anspruch 1.

**8.** N-[2-(Tetrahydro-pyran-4-yloxy)-acetyl]-staurosporin nach Anspruch 1.

**9.** Eine Verbindung nach Anspruch 1 der Formel I, ausgewählt aus
N-[0-(Tetrahydropyran-4-yl)-D-lactoyl]-7-oxo-staurosporin,
N-[O-(Tetrahydropyran-4-yl)-L-lactoyl]-staurosporin,
N-[2-(Tetrahydro-pyran-4-yloxy)-acetyl]-7-oxo-staurosporin,
N-[2-(Tetrahydropyran-4-yloxy)-acetyl]-7-hydroxy-staurosporin und
N-[2-Methyl-2- (tetrahydropyran-4-yloxy)-propionyl]-staurosporin.

**10.** Eine Verbindung der Formel I nach einem der Ansprüche 1-9 zur Anwendung in einem Verfahren zur therapeutischen Behandlung des menschlichen oder tierischen Körpers.

**11.** Pharmazeutische Präparate, die eine Verbindung der Formel I nach einem der Ansprüche 1-9 zusammen mit pharmazeutischem Trägermaterial enthalten.

**12.** Verwendung einer Verbindung der Formel I nach einem der Ansprüche 1-9 zur Herstellung von pharmazeutischen Präparaten, die für die Anwendung zur Behandlung von Krankheiten bestimmt sind, welche auf eine Hemmung der Proteinkinase C ansprechen.

**13.** Verfahren zur Herstellung einer Verbindung der Formel I nach Anspruch 1, dadurch gekennzeichnet, dass man ein Amin der Formel II,

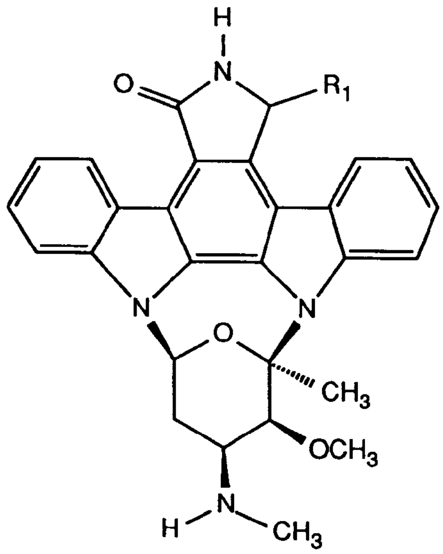

(II)

worin $R_1$ Wasserstoff, Hydroxy, Niederalkoxy oder Oxo bedeutet mit der Massgabe, dass eine durch $R_1$ repräsentierte Hydroxygruppe erforderlichenfalls durch eine leicht abspaltbare Hydroxyschutzgruppe geschützt ist, mit einer Carbonsäure der Formel III,

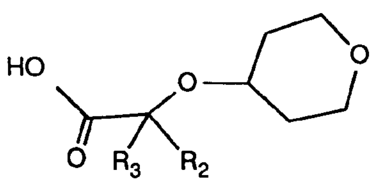

(III)

worin $R_2$ Wasserstoff oder $C_{1-4}$-Alkyl und $R_3$ Wasserstoff oder $C_{1-4}$-Alkyl bedeuten, oder einem reaktionsfähigen Carbonsäurederivat davon acyliert, und Schutzgruppen, welche im gewünschten Endprodukt der Formel I nicht vorhanden sind, abspaltet und, wenn erwünscht, ein erhaltenes Isomerengemisch auftrennt.

**14.** Tetrahydropyran-4-yloxy-alkansäuren der Formel III,

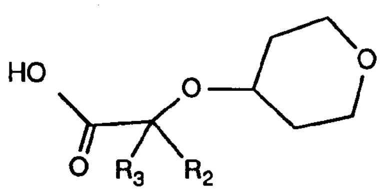

(III)

worin $R_2$ Wasserstoff oder $C_{1-4}$-Alkyl und $R_3$ Wasserstoff oder $C_{1-4}$-Alkyl bedeuten.

Europäisches Patentamt

# EUROPÄISCHER RECHERCHENBERICHT

Nummer der Anmeldung
EP 94 81 0239

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | EP-A-0 532 178 (IMPERIAL CHEMICAL INDUSTRIES)<br>* Seite 52; Beispiel 116 * | 14 | C07D498/22<br>C07D309/12<br>A61K31/55<br>//(C07D498/22, 311:00,273:00, 209:00,209:00, 209:00) |
| A | EP-A-0 383 919 (KYOWA HAKKO KOGYO)<br>* Seite 1; Anspruch 1 * | 1,11 | |
| A | EP-A-0 296 110 (CIBA-GEIGY)<br>* Ansprüche 1,24 * | 1,11 | |

RECHERCHIERTE SACHGEBIETE (Int.Cl.5)

C07D
A61K

Der vorliegende Recherchenbericht wurde für alle Patentansprüche erstellt

| Recherchenort | Abschlußdatum der Recherche | Prüfer |
|---|---|---|
| DEN HAAG | 15. Juli 1994 | Voyiazoglou, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)